# EUROPEAN PATENT SPECIFICATION

(11) **EP 3 958 729 B1**
(45) Date of publication and mention of the grant of the patent: **02.07.2025**
(21) Application number: 20723804.9
(22) Date of filing: 23.04.2020
(51) Int. Cl.: A61B 5/00, A61B 5/08, A61B 5/113, G01N 21/47, A61B 1/267

(54) **A DEVICE FOR MONITORING A PULMONARY SYSTEM OF A SUBJECT**
VORRICHTUNG ZUR ÜBERWACHUNG EINES LUNGENSYSTEMS EINES PATIENTEN
DISPOSITIF DE SURVEILLANCE D'UN SYSTÈME PULMONAIRE D'UN SUJET

(30) Priority: 26.04.2019 SE 1950510
(43) Date of publication of application: 02.03.2022
(73) Proprietor: Neola Medical AB, 223 63 Lund (SE)
(72) Inventor: FELLMAN, Vineta, 00170 Helsingfors (FI); LARSSON, Marcus, 237 34 Bjärred (SE); KRITE SVANBERG, Emilie, 224 65 Lund (SE); LARSSON, Jim, 226 47 Lund (SE); LEANDER, Dennis, 211 29 Malmö (SE); BERGSTEN, Sara, 224 68 Lund (SE); LEWANDER XU, Märta, 224 65 Lund (SE)
(74) Representative: KIPA AB
(86) International application number: PCT/EP2020/061326
(87) International publication number: WO 2020/216839

(56) References cited:
- EP-A1- 3 338 077
- EP-A1- 3 338 077
- US-A- 5 005 573
- BEEK J F ET AL: "Physics in Medicine & Biology", 30 June 1997 (1997-06-30), XP093266908, Retrieved from the Internet <URL:https://iopscience.iop.org/article/10.1088/0031-9155/42/11/018/pdf>
- EMILIE KRITE SVANBERG ET AL: "Diode laser spectroscopy for noninvasive monitoring of oxygen in the lungs of newborn infants", PEDIATRIC RESEARCH, vol. 79, no. 4, 1 April 2016 (2016-04-01), US, pages 621 - 628, XP055311242, ISSN: 0031-3998, DOI: 10.1038/pr.2015.267
- MÄRTA LEWANDER ET AL: "Nonintrusive gas monitoring in neonatal lungs using diode laser spectroscopy: feasibility study", JOURNAL OF BIOMEDICAL OPTICS, vol. 16, no. 12, 1 January 2011 (2011-01-01), pages 127002, XP055708981, ISSN: 1083-3668, DOI: 10.1117/1.3663211

## Description

### BACKGROUND OF THE INVENTION

### Field of the Invention

This disclosure pertains to examining gas filled cavities in a body of a human using an optical device. Especially, the disclosure relates to a device for monitoring a pulmonary system.

### Description of the Prior Art

The pulmonary transition from intrauterine environment to normal postnatal air breathing may cause pulmonary diseases that are increasingly prevalent with decreasing gestational age of the newborn infant. With increasing survival of extremely preterm born infants the number of infants needing respiratory support in neonatal care units will increase. Abnormal pulmonary aeration is common in postnatal lung diseases, such as respiratory distress syndrome due to surfactant deficiency and bronchopulmonary dysplasia. Despite modern respiratory support methods and minimally invasive surfactant therapy, there is still a risk of a life-threatening complication, such as pneumothorax.

Presently, lung aeration problems are partially diagnosed with pulmonary X-ray, which unfortunately only gives a snap-shot of the current condition and when repeated causes a load of harmful radiation, increasing the risk of malignancy development later in life.

Another method is positive transillumination where doctors shine a strong light through the affected side of the newborn's chest while in a darkened room. This procedure is done to show free air in the area surrounding the lungs (pleural cavity). Positive transillumination is analysed visually and is not used for continuous monitoring of the pulmonary system.

Therefore, there is an evident clinical need to be able to monitor the gas content in the lungs and to improve detection of pulmonary complications, while, as much as possible, avoiding ionizing radiation. It may also be an advantage if the method may include rapid detection of life-threatening complications such as pneumothorax and obstruction of bronchi causing varying degree of atelectasis.

Hence, new improved apparatuses and methods for monitoring a pulmonary system, in particular for detecting lung aeration problems, are advantageous.

Diode laser spectroscopy for noninvasive monitoring of oxygen in the lung of newborn infants; Emilie Krite Svanberg et al; Pediatric RESEARCH, Vol 79 number 4, April 2016 describes a method and system for measuring concentration of oxygen gas and water vapor in the lung using laser spectroscopy.

EP3 338 077 A1 describes a device and system for measuring free gas in a cavity of a subject and a control unit for evaluating the detected transmitted light for determining a concentration of the free gas.

### SUMMARY OF THE DISCLOSURE

Accordingly, embodiments of the present disclosure preferably seek to mitigate, alleviate, or eliminate one or more deficiencies, disadvantages, or issues in the art, such as the above-identified, singly or in any combination by providing a device, system, or method according to the description for measuring free gas in a cavity, such as a lung. The device, system, or method are adapted for monitoring a pulmonary system and to detect a status of the pulmonary system, such as detecting aeration problems, such as lung aeration problems and/or pulmonary complications, more particularly diagnosis of pneumothorax and/or atelectasis.

The invention is as defined by the appended claims.

It should be emphasized that the term "comprises/comprising" when used in this specification is taken to specify the presence of stated features, integers, steps or components but does not preclude the presence or addition of one or more other features, integers, steps, components or groups thereof.

### BRIEF DESCRIPTION OF THE DRAWINGS

These and other aspects, features and advantages of which examples of the disclosure are capable of will be apparent and elucidated from the following description of examples of the present disclosure, reference being made to the accompanying drawings, in which:
Fig. 1 is illustrating an example of a disclosed device and system;
Figs. 2A to 2D are illustrating examples of arrangements of a light source in the trachea for measuring lung functions;
Fig. 3 is illustrating a further example of an arrangement of a light source using a bronchoscope for measuring lung functions;
Fig. 4A and 4B are illustrating further examples of an arrangement wherein a light source is inserted through the oesophagus;
Fig. 5 shows time evolution of O2 absorption signal and corresponding light transmission for induced atelectasis in four piglets; and
Fig. 6 shows time evolution of O2 absorption signal and corresponding light transmission for induced pneumothorax in four piglets.

### DESCRIPTION OF EXAMPLES

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art.

The following disclosure focuses on examples of the present disclosure applicable for monitoring a pulmonary system of a subject, for example by arranging a light source or an optical member in a cavity, such as in the trachea, or in the digestive system, such as in the oesophagus or the intestines, such as parts of the digestive tract and detecting the transmitted light using a detector, for example one or a plurality of detectors arranged outside the human body.

For example, this is advantageous for detecting weak signals due to the light passing a long path through the tissue enclosing the cavity.

Alternatively, both the detector and the light source may be arranged dermally. The light source is then configured to transmit light to the cavity and the detector is configured for detecting light scattering back which has passed through the cavity at least once. Such arrangements are described in EP1871221.

A light signal, such as an absorption spectrum, related to the free gas may be used for monitoring a pulmonary system to determining a status of the pulmonary system, such as a physiological status of the pulmonary system. Physiological problems may, for example, be aeration problems and/or pulmonary complications, such as lung aeration problems, more particularly diagnosis of pneumothorax and/or atelectasis.

In some examples of the disclosure, the light source is arranged to transmit light to the tissue with losses as low as possible and then detect the light at the surface of the skin. By arranging the light source or optical member internally for decreasing the attenuation factor of the light transmitted to the tissue for measuring the free gas in a cavity, the total attenuation factor may be reduced to about 0.001 which is about 1000 times larger than what was previously be achieved.

In some examples, a free gas, or a mixture of gases, in a cavity of the pulmonary system may be monitored for determining the status of the pulmonary system, such as the physiological status of the pulmonary system. For obtaining the concentration and/or distribution of the free gas, GASMAS technology may be applied.

When arranging the light source internally, the disclosed devices, systems and methods utilize a member for introducing the light sources for injecting the light into the tissue, for example, a bronchoscope, a nasogastric feeding tube, endoscope, a tracheal tube, a colonoscope or similar introducing members.

One group of patients considered to be relevant when it comes to monitoring the pulmonary system are preterm born infants. These infants often experience respiratory complications, such as respiratory distress syndrome, breathing difficulties and problems with the lung functioning. Preterm born infants are therefore often connected to a medical ventilator to assist them with moving air into and out of the lungs. When connected to a medical ventilator, a preterm born infant is intubated with an endotracheal tube. In one example of the description it is described how a fibre optical arrangement for delivering light to a cavity, such as the lung, may be conducted in combination with a tracheal tube, such as an endotracheal tube while detection of transmitted light is made at the skin surface.

In another example of the description it is described how a fibre optical arrangement for delivering light to a cavity, such as the lung, may be conducted in combination with a nasogastric feeding tube inserted into the oesophagus while detection of transmitted light is made at the skin surface.

Additionally, in another example, the light from the optical fibre arrangement is distributed over a larger distance or area by a light diffusing material. Additionally, and/or alternatively, the transmitted light is detected at the skin surface by one or a plurality of detectors positioned at the skin over the cavity such as the lung. The detectors may be configured to be arranged at the skin surface or other means, such as optical fibres, could be used to collect the light at the skin surface and direct the collected light to a detector. In one example, a plurality of detectors may be used in parallel or sequentially to detect gases or a distribution of gases, or gas concentrations in different parts of the bronchial tree or lung of a patient.

In this way, the results of changes in the settings of the medical ventilator or medications may be directly observed, and the information from the observation may be used to optimize the treatment of the patient using a feedback system.

In some examples, the detection is made frequency- and/or phase-sensitively. The light, such as laser light, from the light source may be wavelength modulated at a selected frequency, and synchronous intensity variations may be detected when the modulation is conducted around a gas absorption wavelength. When modulation is conducted close to a gas absorption wavelength, the intensity of the detected light will quickly change at small variations of the wavelength, as described in S. Svanberg, Gas in Scattering Media Absorption Spectroscopy - from Basic Studies to Biomedical Applications, Lasers and Photonics Reviews 7, 779 (2013).

Alternatively, in some examples, a skin area may be detected by an imaging sensor, such as a digital camera, with high intensity dynamic both at an absorbing wavelength and at a close non-absorbing wavelength. The two images may then be compared, for example by division or subtraction, whereby the areas affected by gases may be visualized.

When utilizing the GASMAS technology, the determination of a gas concentration is affected by the path length within the gas filled cavity that the light interacting with the gas has to travel through. The path length in the tissue is unknown due to multiple scattering. Hence light travelling both longer and shorted distances through the tissue may be detected at the same time. Beer-Lambert's relation which is normally used when analysing gas gives that the intensity of the absorption signal is determined by the product of the gas concentration and the path length (see S. Svanberg, Atomic and Molecular Spectroscopy - Basic Aspects and Practical Applications, 4th Edition, Springer, Berlin, Heidelberg 2004. When the path length is known, which is the case when measuring in a non-scattering material, the gas concentration may be directly calculated.

When utilizing the GASMAS technology for measuring a gas concentration, the path length through the gas is unknown; this need to be accounted for when performing a gas concentration measurement. Different method may be used for handling the problems of an unknown path length, these are discussed in, for example L. Mei, G. Somesfalean and S. Svanberg, Pathlength Determination for Gas in Scattering Media Absorption Spectroscopy, Sensors 14, 3871 (2014)

One of the most exact methods is to use profile changes in an absorption spectrum of, for example, a water vapour absorption line. The changes in the profile of the water vapour line are dependent on the oxygen concentration, see P. Lundin, L. Mei, S. Andersson-Engels and S. Svanberg, Laser Spectroscopic Gas Concentration Measurements in Situations with Unknown Optical Path Length Enabled by Absorption Line Shape Analysis, Appl. Phys. Lett. 103, 034105 (2013). This method requires a good signal to noise ratio since the influence of the oxygen on the water vapour line is weak.

Another option is to perform the GASMAS measurements both for the gas concentration to be determined and for water vapour. The water vapour concentration may be assumed to be saturated in tissue and wherein the concentration is determined by the temperature, which is known, see A.L. Buck, Buck Research Manuals; Updated Equation from (1981). New Equation for Computing Vapor Pressure and Enhancement Factor. J. Appl. Meteorol. 20, 1527 (1996). Based on the measured water vapour signal the effective path length for the wavelength used may be calculated. As long as the difference between the wavelengths used is small, the obtained path length may be approximately the same as for light being transmitted through the gas with an unknown concentration to be determined, such as oxygen, nitric oxide (NO), or carbon dioxide. The gas concentration, such as for oxygen, nitric oxide (NO), and carbon dioxide, may then be directly calculated using the approximated path length. This method works best when the light absorption and the light scattering in the tissue is the same for both measurements which is the case when the wavelengths used for the measurements are close. For example, oxygen is normally monitored around some of the sharp components in the oxygen molecule's A-band about 760 nm. Water vapour has a strong absorption around, for example 935 nm, but the difference in wavelength compared to oxygen may need to be corrected for due to differences in the optical properties at the different wavelengths. Therefore, the weaker absorption wavelength for water vapour at around 820 nm may be a better choice.

In preferred examples, the device, system, and method described herein, are utilizing Tunable Diode Laser Absorption Spectroscopy (TDLAS) to detect gas by multiple light scattering in tissue where the absorbing free gas is dispersed in cavities surrounded by a medium. In TDLAS, the wavelength of the light source is swept over an absorption peak or band. A sweep where each step could be down to a scale below a nanometre.

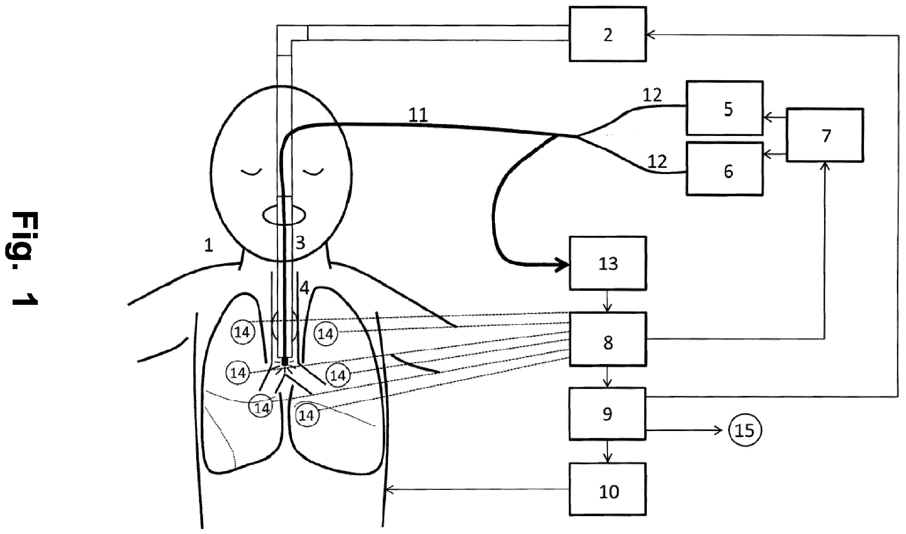

One example of an implementation is illustrated in Fig. 1. The patient 1 in this example is a preterm born infant. The infant is connected to a medical ventilator 2 due to, under the circumstances common disorder, respiratory distress syndrome (RDS).

The medical ventilator 2 is connected to the intubated patient 1 via, for example, an endotracheal tube 3 inserted in the trachea 4. In this example, two light sources 5, 6 are used for measuring a gas, for example oxygen at about 760 nm, and water vapour at about 820 nm. Depending on the measured gas other wavelengths may be used. In some examples other gases than water vapour may be used as a reference gas. The requirement is only that the gas concentration may be calculated without using the path length the detected light has travelled.

Alternatively, in other examples only one light source may be used. In some other examples more than two light sources may be used for detecting further gases, gas distributions or gas concentrations.

Alternatively, other configurations of the light sources may be possible when using other methods related to GASMAS as previously described herein.

The light sources may be semiconductor lasers, for example distributed feed-back lasers (DFBL), vertical cavity surface emitting lasers (VCSEL) or other types of available lasers. The power of the emitted light is preferably in the range 1mW to 3000mW.

The lasers may be driven by a current and temperature regulating unit included in the drive unit 7. The drive unit 7 may be controlled by a control unit 8, such as a computer. The control unit 8 may be used for signal processing and evaluation of the measured data.

All determinations or calculations described herein may be performed by a control unit or a data processing device (not illustrated).

The control unit or a data processing device may be implemented by special-purpose software (or firmware) run on one or more general-purpose or special-purpose computing devices. In this context, it is to be understood that each "element" or "means" of such a computing device refers to a conceptual equivalent of a method step; there is not always a one-to-one correspondence between elements/means and particular pieces of hardware or software routines. One piece of hardware sometimes comprises different means/elements. For example, a processing unit serves as one element/means when executing one instruction, but serves as another element/means when executing another instruction. In addition, one element/means may be implemented by one instruction in some cases, but by a plurality of instructions in some other cases. Such a software controlled computing device may include one or more processing units, e.g. a CPU ("Central Processing Unit"), a DSP ("Digital Signal Processor"), an ASIC ("Application-Specific Integrated Circuit"), discrete analogue and/or digital components, or some other programmable logical device, such as an FPGA ("Field Programmable Gate Array"). The data processing device 10 may further include a system memory and a system bus that couples various system components including the system memory to the processing unit. The system bus may be any of several types of bus structures including a memory bus or memory controller, a peripheral bus, and a local bus using any of a variety of bus architectures. The system memory may include computer storage media in the form of volatile and/or non-volatile memory such as read only memory (ROM), random access memory (RAM) and flash memory. The special-purpose software may be stored in the system memory, or on other removable/non-removable volatile/non-volatile computer storage media which is included in or accessible to the computing device, such as magnetic media, optical media, flash memory cards, digital tape, solid state RAM, solid state ROM, etc. The data processing device 10 may include one or more communication interfaces, such as a serial interface, a parallel interface, a USB interface, a wireless interface, a network adapter, etc., as well as one or more data acquisition devices, such as an A/D converter. The special-purpose software may be provided to the control unit or data processing device on any suitable computerreadable medium, including a record medium and a read-only memory.

Additionally, in some examples, the control unit 8 may be connected to the controller 9 of the medical ventilator 2 for controlling the settings of the medical ventilator 2.

Additionally, and/or alternatively, the controller 9 may also be used to control the distribution of medicaments to the patient 1.

Additionally, in some examples, the lasers may be wavelength modulated by modulating the drive current on two separate frequencies. The frequencies may typically be in the region around 10kHz to allow noise reduced phasesensitive detection (lock-in-detection).

By using separated modulation frequencies, different gases, such as oxygen, nitric oxide (NO), and carbon dioxide, and water vapour, may be separated even though the light injection may be carried out through the same fibre 11. Light from individual optical fibres 12 connected to the light sources, such as the semiconductor lasers 5, 6, may be connected to a single injection fibre 11.

Additionally, in some examples, a small portion of the light to be injected may be diverted optically, such as 5 by a fibre, to a calibration unit 13. The calibration unit 13 may be a gas cell including a gas to be detected, such as oxygen, nitric oxide (NO), and carbon dioxide. The gas has a known concentration and the gas cell has a predetermined length. The calibration unit 13 may also include a droplet of water and a temperature measuring unit. All parts of the calibration unit 13 may have a common detector unit.

Alternatively, in some examples, a compact diffuse multi-pass cell made from a porous material, such as ceramic may be used. The porous material may be enclosed in a compact gas cell, see T. Svensson, E. Adolfsson, M. Lewander, C.T. Xu and S. Svanberg, Disordered, Strongly Scattering Porous Materials as Miniature Multi-pass Gas Cells, Phys. Rev. Lett. 107, 143901 (2011).

The main part of the individually frequency marked light is led through optical fibre 11 down through the, in this example used, endotracheal tube 3.

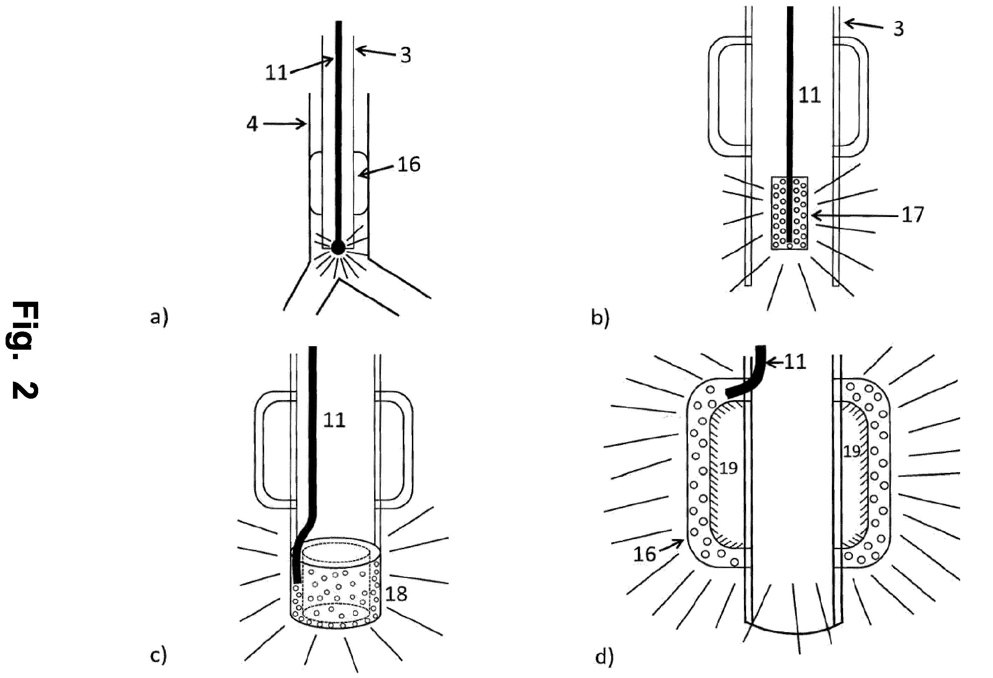

In the example illustrated in Fig. 2B to 2D, the fibre 11 ends with a diffusor. The diffusor may be a structure on the surface of the fibre or a separate part made from a light scattering material. The diffusor is used to have the light distributed over a larger tissue surface to reach a decreased intensity. A lower intensity may help to avoid raise in temperature of the tissue. Another advantage is eye safety if tests are performed outside the human body.

In some examples, the injected light transmitted from the end of the optical fibre 11, such as through the diffusor, may be transmitted to the tissue without passing any air in the trachea. If the light passes through air, the air may give some background signal in light detected by the detectors 14.

In one example, an inflatable cuff or balloon made from optically scattering material and with reflecting material on the inner walls may be used to have as much light as possible to be directly transmitted into the tissue, as seen in Fig. 2A.

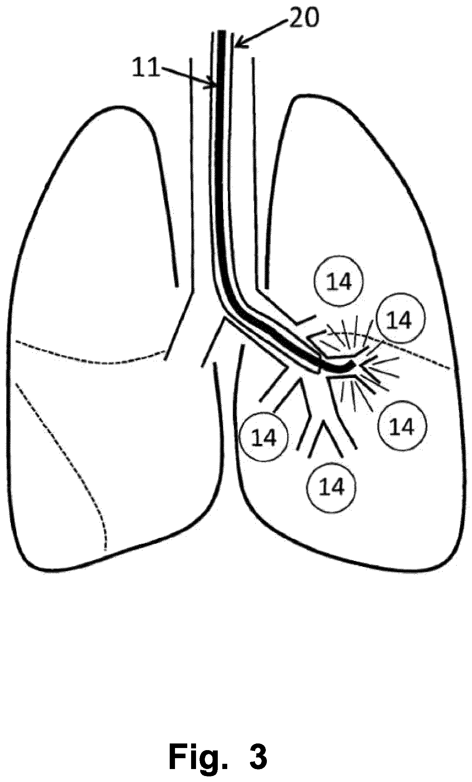

The example illustrated in Fig 2A gives good positioning of the optical fibre for monitoring the upper part of the lungs. By using a bronchoscope and a diffusing fibre end, the light injection may be made deeper into the bronchial tree as illustrated in Fig. 3.

Alternatively or additionally, in some examples more than one, such as at least two, positions are used for light injection. When using more than one location for measuring, the measurements for the different locations need to be performed sequentially. Alternatively, more than one controllable standard fibre may be used.

When using more than one location for light injection and to obtain a better three-dimensional gas distribution analysis, diffuse light tomography may be used, as described in the articles J. Swartling, J. Axelsson, S. Svanberg, S. Andersson-Engels, K. Svanberg, G. Ahlgren, K.M. Kälkner and S. Nilsson, System for Interstitial Photodynamic Therapy with On-line Dosimetry - First Clinical Experiences of Prostate Cancer, J. Biomed. Optics 15, 058003 (2010); and T. Durduran, R. Choe, W.B. Baker and A.G. Yodh, Diffuse Optics for Tissue Monitoring and Tomography, Rep. Progr. Phys. 73, 076701 (2010)

The detector or detectors 14 is/are adapted to be arranged against the skin. The detector should have a surface sized to detect the light transmitted through the tissue, for example in the range 0.25cm² to 5cm², such as 1cm². The detector may be made from different materials, such as germanium.

The detected light is transmitted as an electrical signal to the control unit 8. The signal may be evaluated using digital lock-in technology sequentially or in parallel, as described in the article L. Mei, and S. Svanberg, Wavelength Modulation Spectroscopy - Digital detection of Gas Absorption Harmonics based on Fourier Analysis, Applied Optics 54, 2254 (2015).

Alternatively, in some examples an analogue lock-in-amplifier may be used. The analogue lock-in-amplifier may be connected to the control unit 8.

Additionally, in some example threshold values may be selected. When the measured value has reached or passed the selected threshold values an alarm 15 may be activated for the health care personnel. The alarm 15 may be an acoustic alarm and/or an electronic alarm to a surveillance centre.

Figs. 2A to 2D are illustrating different examples of how to utilise a tracheal tube, such as an endotracheal tube.

Fig. 3 is illustrating an example wherein diffused light is injected using a working channel of a bronchoscope.

It may be observed that the same equipment, with some modification, may be used for external injection of light into the human body in those cases wherein a medical ventilator with a tracheal tube, or a bronchoscope is not used, for example through a feeding tube through the oesophagus. In these cases, the light may be expanded and made diffused by a scattering medium with a large enough surface, for example a few cm², made in contact with the skin. This arrangement makes it possible to avoid local increase in tissue temperature, and eye safety is achieved.

Fig. 1 is illustrating an exemplary configuration of the disclosed device and system. Patient 1 is connected to a medical ventilator 2 via an introducing member 3, such as a bronchoscope, a tracheal tube, or an endotracheal tube, connected to the trachea 4.

In some other examples the introducing member 3 may be, for example, a nasogastric feeding tube.

The light sources 5, 6, such as lasers, with a wavelength associated to the free gas of interest, for example, oxygen, nitric oxide (NO), and carbon dioxide, and a reference gas, for example water vapour.

In some examples, other gases than water vapour may be used as a reference gas. The requirement is only that the gas concentration may be calculated without using the path length the detected light has travelled.

Alternatively, in other examples, only one light source may be used. In some other examples more than two light sources may be used for detecting further gases, gas distributions or gas concentrations.

Alternatively, other configurations of the light sources may be possible when using other methods related to GASMAS as previously described herein.

The light sources are connected to the drive unit 7, which is controlled by the control unit 8.
In some examples, the measured value of the free gas in the lungs may be used for affecting the controller 9 for controlling the setting of the medical ventilator 2.

Alternatively, and/ or additionally, in some examples, the measured value by the control unit 8 may be used for administration of a medicament 10.

The light is emitted to the tissue via an optical fibre 11. Light from individual optical fibres 12 connected to the light sources 5, 6, such as the semiconductor lasers, may be connected to a single fibre 11.

Additionally, in some examples, a small portion of the light to be injected may be diverted optically, such as by a fibre, to a calibration unit 13.

A detector 14 is configured to be positioned at a skin location at the chest of the patient for detecting the transmitted diffused light. The detected light carries information about the gas concentration in the lungs or gas distribution in the lung tissue, such as oxygen, nitric oxide (NO), and carbon dioxide concentration or distribution.

Additionally, in some examples, an alarm 15 may be activated when the measured value reaches or passes a selected threshold value.

Figs. 2A to 2D are illustrating different examples of coupling the light from the optical fibre to the tissue for measuring lung function.

Fig. 2A is illustrating an example of a trachea tube 3, such as an endotracheal tube, with a balloon or cuff 16 being inflatable to prevent air leakage next to the trachea tube 3 which includes the optical fibre 11 for transmitting light down the trachea.

Fig. 2B is illustrating an example of an optical fibre 11 brought down a trachea tube, such as an endotracheal tube, to its end. A light diffusor 17 is arranged as an ending to the optical fibre 11.

Fig. 2C is illustrating an example of how light in the fibre 11 may be connected to the end section 18 of a trachea tube, such as an endotracheal tube. The end section is made from a non-absorbing, but strongly light scattering, material.

Fig. 2D is illustrating an example of how light from an optical fibre 11 may be coupled to a balloon or cuff 16. The balloon or cuff may be made from a non-absorbing, but light scattering, material. In some examples, the inner walls are coated 19 with a light reflecting material.

In an example, the laser light source is applied via the oesophagus rather than the trachea. In this example, the laser light source may be combined with the nasogastric feeding tube that is used in most infants in neonatal intensive care. The use of the nasogastric feeding tube for light application is advantageous because most infants already need to have such a device inserted, so no additional device needs to be introduced. It is also advantageous since the oesophagus environment is less sensitive to infection. It is also advantageous due to that the oesophagus is normally mostly collapsed, so there is potentially not a need for an expanding cuff to create good optical contact between the light source and the tissue. Further, lower parts of the lungs may also be more easily reached.

The light source should be positioned at a point in the oesophagus that represents a position close to the lungs. In an example, an optical fibre that guides the laser light is combined with the nasogastric feeding tube, so that the optical fibre goes along the tube to an adequate position along the tube. Nasogastric feeding tubes have marks that indicate how deep the tube is inserted, and these marks can be used to determine the position of the light source in the oesophagus.

In a preferred example, the optical fibre that guides the laser light is embedded in the tube wall during manufacturing of the nasogastric feeding tube, so that the nasogastric feeding tube and laser light guide comes as a single device.

In an example, the distal end of the optical fibre is terminated with a light diffusor that distributes the light over a larger area than would be provided by the fibre tip only. In a preferred example, when the optical fibre is embedded in the tube wall as described above, the diffusor is implemented by designing the tube wall in front of the fibre tip with light scattering properties so that the light scatters over an area along the tube corresponding to the desired characteristics of the diffusor. In an alternative example, the diffusor is manufactured as a separate component that is embedded in the tube wall similarly to the optical fibre.

In an alternative example, the diffusor can be made part of an expandable cuff similar to the diffusor described in connection with the trachea herein above.

The optical fibre may also be positioned in the nasogastric tube in such a way, that it may be sequentially moved to different positions along the tube for facilitating, e.g. a full tomographic view of the gas distribution, such as oxygen, nitric oxide (NO), and carbon dioxide distribution, using multiple detectors 14.

Alternatively, a plurality of optical fibres may be used in parallel at different positions for facilitating, e.g. a full tomographic view of the gas distribution, such as oxygen, nitric oxide (NO), and carbon dioxide distribution, using multiple detectors 14.

In alternative examples, the light source on the nasogastric feeding tube is implemented by placing one or multiple laser diodes directly at the site where the light source should be and having electrical wires for driving the laser diodes along the nasogastric feeding tube. This implementation may also be applied to the cases of a tracheal tube.

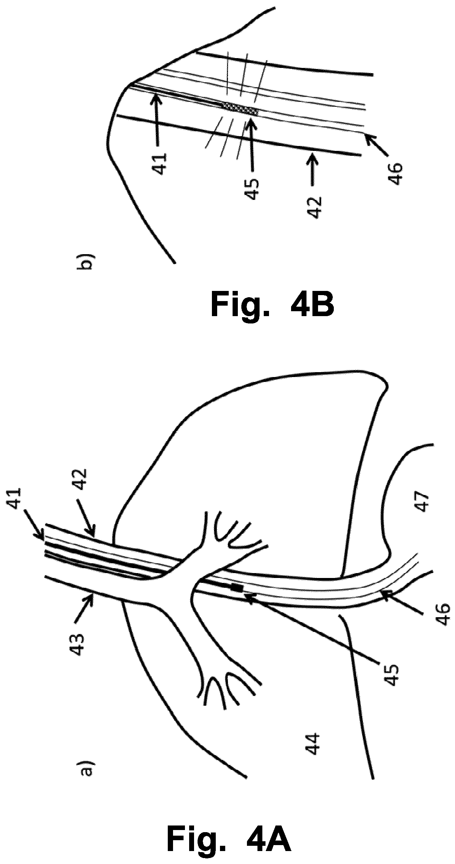

Figure 4A illustrates an optical fibre 41 combined with a nasogastric feeding tube 46 inserted into the oesophagus 42. The relation with the trachea 43 and the lungs 44 is also shown. At the distal end of the optical fibre 41, there may be a light diffusor 45. The upper part of the stomach 47 is also shown.

Figure 4B illustrates a close-up of the optical fibre 41, embedded into the tube wall of the nasogastric feeding tube 46. The nasogastric feeding tube is inserted in the oesophagus 42. In this example, the optical fibre has a diffusor 45 at the distal end of the optical fibre 41. In other examples, the optical fibre 41 may not have a diffusor.

Alternatively, the gas concentration could be assessed noninvasively using light being transmitted from outside the human, such as towards the skin over the cavity containing the free gas, such as the lungs. The light that has penetrated enough tissue to reach the cavity is scattered and transmitted to a detector positioned against the skin. For example, the detector is typically positioned a few centimetres away from the position of the light source. A non-invasive measurement procedure may thereby be performed.

The same arrangement, with the light source arranged dermally, may be used when only detecting and evaluating a transmission signal for determining the status of the pulmonary system, or when correlating transmission and gas concentration and/or distribution measurements.

In the examples above, the light source or optical member is described as an optical fibre arrangement connected to a light source, such as a laser, but other arrangements are equally possible. For example, may the light source or optical member be a waveguide, or an LED directly arranged in the introducing member.

When arranging a light probe on a skin surface, i.e. dermal, a fibre arrangement connected to a light source may be used but a light source may, in some examples, be adapted to be directly arranged on the skin surface, such as a LED, laser, and waveguide.

The detector unit may be photodiodes, photomultiplier tubes, avalanche photodiodes, charge-coupled devices, CCD or CMOS light sensitive devices. As described herein, the detector may be a single detector or could be a plurality of detectors.

Different ways of measuring free gas in a cavity have been described above, these may be used for monitoring the pulmonary system and for determining a status of the pulmonary system, such as detecting aeration problems and/or pulmonary complications, such as lung aeration problems, more particularly diagnosis of pneumothorax and/or atelectasis.

The same techniques described for introducing a light signal to a cavity of the pulmonary system and detecting light transmitted through for observing a concentration and/or distribution of a free gas in the cavity, may be used for measuring and evaluating an intensity of the transmitted light. The determination of the status of the pulmonary system may be performed by evaluating changes to the intensity of the transmitted light, such as over time, or by evaluating the shape of the intensity curve over time.

When evaluating the transmitted light for determining the status of the pulmonary system, such as detecting aeration problems and/or pulmonary complications, such as lung aeration problems, more particularly diagnosis of pneumothorax and/or atelectasis, the wavelength could be any wavelength transmittable through the tissue surrounding the cavity and still detectable. The wavelength is within the optical window for tissue, which will give a larger penetration depth. Additionally, in some examples, the signal is associated with the same wavelength over time. This means that the wavelength is not swept but kept constant. For example, by keeping the light source locked at a single wavelength. This wavelength used only when evaluating the intensity signal may not be associated with an absorption band of a free gas. This is a difference compared to measuring a concentration or distribution of at least one gas when the wavelengths used need to be associated with the absorption of the at least one gas. The term "single wavelength" should be interpreted as the central wavelength of the emitted light from the light source and covers that the emitted light has a linewidth.

Changes over time in an intensity of the detected light signal, transmitted through a cavity, may be associated with a variation of a volume of the cavity over time. This is related to that the intensity of the detected signal may change due to the distance the light travels through the cavity. This could be used as a qualitative measure of the aeriation of a cavity, such as a lung. For example, by monitoring or observing the variation over time in the intensity of the light signal transmitted through the cavity, an increase and/or decrease in the intensity may be associated with an increase and/or decrease in volume of the cavity.

The volume of different cavities may also be compared, for example by obtaining a first transmission signal of a first cavity, such as a first lung, and a second transmission signal of a second cavity, such as a second lung. The first and second transmission signal may then be compared to detect a change in a volume of one of said cavities in relation to the other cavity.

Detecting or monitoring variations in a transmission signal due to changes of a volume may be used to detect issues with aeriation of lungs. Changes in the volume could, for example, mean that the tube used for aeriation is not positioned correctly or has been blocked. Variations in the transmission signal due to variations in the volume may be used to detect an overpressure in at least one lung or a collapse, such as a partial collapse of at least one lung. It may also be used to detect an inflammation or sepsis in the lungs. Detection of volume changes may also be used to detect problems with increased mucus in the lungs.

The determination of a status of the pulmonary system may be done by continuously monitoring a signal transmitted through a cavity of the pulmonary system, such as a lung. Determining the status, such as detecting aeration problems and/or pulmonary complications, could be done by evaluating a detected light signal transmitted through the lung. A pulmonary complication and/or an aeration problem may be detected by observing changes in the detected light signal, such as an increase or a decrease in the signal over time or changes to an intensity curve of a light signal transmitted through the cavity.

An option may be to obtain the concentration, and/or distribution of a free gas based on a detected light signal transmitted through the cavity of pulmonary system. The light should preferably have a wavelength associated with the absorption band of the free gas to be detected. The concentration and/or distribution could be obtained by using the above described systems. Further, GASMAS technology may also be applied. The free gas may be a physiological gas or a mixture of gases, such as any of oxygen, nitric oxide (NO), carbon dioxide, anaesthesia gases and water vapour.

The status of the pulmonary system, such as pulmonary complication and/or an aeration problem, may be detected by observing changes in the concentration and/or distribution of the free gas, such as an increase or a decrease of the concentration over time, or changes to shape of the concentration curve. The concentration of the free gas may be obtained as an absorption signal of the free gas. The absorption signal may have the dimension [%m] and be the product of the relative gas concentration [%] and the absorption pathlength [m].

A further option may be to monitor the pulmonary system by correlating the detected light transmitted through the cavity of the pulmonary system, such as a lung, and the concentration/distribution signal of the free gas. The emitted light for the evaluation of the intensity of the transmission may have the same wavelength as the emitted light for obtaining the concentration and/or distribution of a free gas in the cavity, such as a wavelength associated with an absorption band of the free gas. Alternatively, the emitted light for the evaluation of the intensity of the transmission may have a different wavelength as the emitted light for obtaining the concentration and/or distribution of a free gas in the cavity. For example, the emitted light for the intensity evaluation may be any wavelength within the optical window for tissue and the emitted light for obtaining the concentration and/or distribution of a free gas may be associated with an absorption band of the free gas.

To show that monitoring the pulmonary system for detecting aeration problems and/or pulmonary complications, such as lung aeration problems is possible, measurements were carried out on four piglets. For the measurements a GASMAS system was used to measure gas absorption at two wavelengths, 764 nm for oxygen gas absorption and 820 nm for water vapor absorption.

The relative humidity in the lung is approximately 100%, and together with knowledge of tissue temperature and air pressure, the water vapor gas concentration can be theoretically calculated. The measured water vapor gas absorption in [%m] thus provides the absorption path length in [m] for light at 820 nm. Assuming that the light at 764 nm travels the same average distance in tissue, the measured oxygen gas absorption in [%m] then provides the oxygen concentration in [%].

The emission from two diode lasers providing the light at 764 nm and 820 nm was focused into an optical fibre. The diode lasers and the light combining optics were enclosed in a nitrogen-filled box to eliminate offsets in the absorption signal.

The gas absorption signal was obtained by sequentially scanning the wavelength of the lasers, at a rate of 1 kHz, over one of the absorption lines from the oxygen molecule (O2) at 764 nm and from water vapor (H2O) at 820 nm. The two gases were measured in sequence with a measurement time of 100s before switching.

Optical fibres were inserted into the piglets for oesophageal light administration and the end of the fibre was configured for diffusing the light.

After propagating through the tissue, the transmitted light was detected by a photodiode incorporated into a detector probe attached to the skin. The detector probe area was covered with a layer of ultrasonic gel in order to reduce offsets in the absorption signal. The detected absorption signal was intensity normalized and frequency filtered by the electronic platform which acquired the absorption magnitude.

A partial pulmonary atelectasis was simulated by inserting a 4 Fr embolectomy catheter with a balloon at its tip (LeMaitre^{®} Vascular, Sulzbach, Germany) into the right main bronchus. Partial obstruction of the airways was considered confirmed when the end-tidal CO₂ was decreased by 50% immediately after balloon inflation. The balloon was deflated after approximately 1.5 minutes or if the piglet became circulatory unstable. Continuous measurement of the oxygen signal was performed during the procedure, which were repeated three times.

After the piglet had recovered from atelectasis, a small thoracostomy was prepared in the right axillary line, 1 cm below the dermal detector probe, and a drainage tube was placed and fixated with suture. The piglet was ventilated with 100% oxygen while 150ml of atmospheric air (21% oxygen) was injected into the pleural space through the tube. The oxygen signal was continuously measured during the pneumothorax phase. Approximately 2 minutes after induction, the air was withdrawn from the pleural cavity and pulmonary recruitment followed. The procedure was repeated three times.

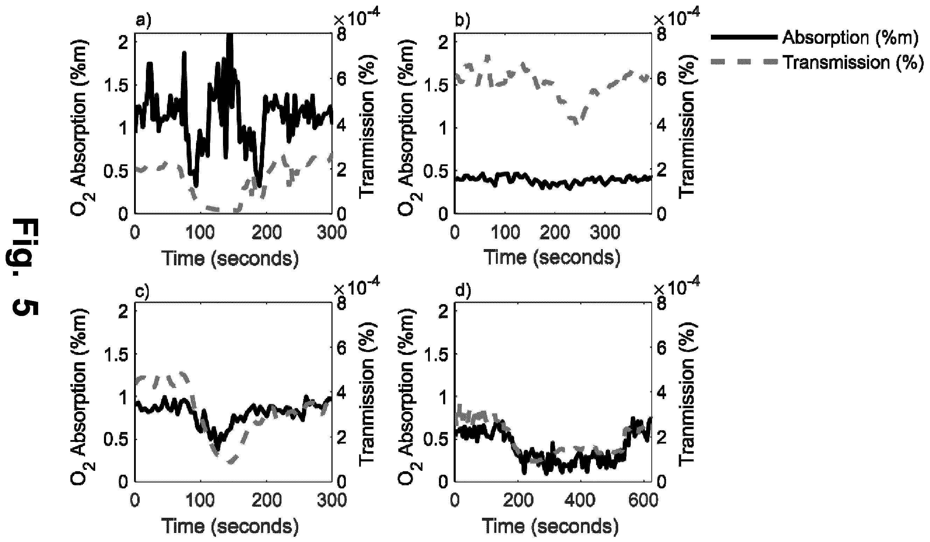

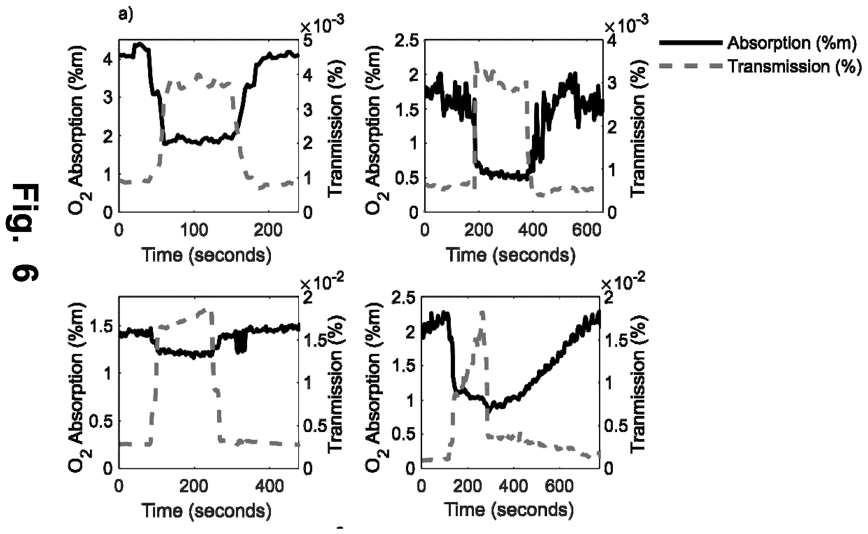

Atelectasis was induced in four of the piglets while continuously monitoring the oxygen absorption and light transmission using the internal light source, see Fig. 5 which shows time evolution of O2 absorption signal and corresponding light transmission for induced atelectasis. A decrease of both oxygen absorption and light transmission at the initiation of atelectasis can be seen. The dashed line shows the transmission signal in percent and the solid line shows the absorption in %m. When the balloon was deflated in the bronchus, the absorption and light transmission was observed to, in most cases, return to the initial value before atelectasis. For the pneumothorax measurements, see Fig. 6, which shows time evolution of O₂ absorption signal and corresponding light transmission for induced pneumothorax, the FiO2 was set to 1.0 and pneumothorax was induced by injecting air into the pleural space. The continuously monitored oxygen absorption decreased when the pneumothorax was initiated. Simultaneously, an increase in light transmission was detected. The dashed line shows the transmission signal in percent and the solid line shows the absorption in %m.

To improve the detection of the detected light signal which has been transmitted through the cavity of the pulmonary system, such as a lung, different methods may be applied. In some examples, an optical filter with a transmission at the wavelength of the emitted light signal may be arranged in front of the detector unit. The optical filter may pass long wavelengths only (long-pass), short wavelengths only (short-pass), or a band of wavelengths, blocking both longer and shorter wavelengths (bandpass), or the optical filter may be a combination thereof. The passband may be narrower or wider; the transition between maximal and minimal transmission can be sharp or gradual. Filters with more complex transmission characteristic may be used, for example with two peaks rather than a single band.

Additionally and/or alternatively, in some examples, the emitted light signal may be pulsed with gated detection. The gated detection may, for example, be timegated, whereby the pulsed light source is pulsed at a frequency, such as at a user-defined frequency. For a period after the light pulse (known as the 'Gate Delay') the camera is insensitive. The camera then becomes sensitive for a time period (known as the 'Gate Width'). Additionally and/or alternatively, in some examples, the emitted light signal may be amplitude modulated. The modulation may be seen in the detected signal and with frequency analysis of the detected signal, the amplitude at the modulation frequency will be a measure of transmission with less influence of noise and background signal. In addition, the frequency of the amplitude modulation may also be varied for further possibilities of noise and background suppression, such as the emitted light signal is amplitude modulated with a varied modulation frequency and the frequency spectrum of the detected signal is analysed at the varying modulation frequency.

In some examples, the determined status of the pulmonary system may be used as a feedback signal to a medical ventilator. For example, the settings of the medical ventilator may be changed in response to the detected status, for example automatically. This could be done by having the control unit to send a signal to the medical ventilator, or to have the control unit as an integrated part in the medical ventilator.

The medical ventilator may here be, for example, an invasive ventilation (conventional), a high-frequency oscillatory ventilation (HFOV), or Continuous Positive Airway Pressure (CPAP). But a medical ventilator could also mean a passive breathing support, such as high flow nasal cannula (HFNC).

One setting that may be changed is the pressure. The control unit may, for example, be configured to send a signal to the medical ventilator to increase the pressure of when an atelectasis is detected, and/or to reduce the pressure when atelectasis is reduced.

The present invention has been described above with reference to specific examples. However, other examples than the above described are equally possible within the scope of the disclosure. Different method steps than those described above, performing the method by hardware or software, may be provided within the scope of the invention. The different features and steps of the invention may be combined in other combinations than those described. The scope of the disclosure is only limited by the appended patent claims.

The indefinite articles "a" and "an," as used herein the specification and in the claims, unless clearly indicated to the contrary, should be understood to mean "at least one." The phrase "and/or," as used herein the specification and in the claims, should be understood to mean "either or both" of the elements so conjoined, i.e., elements that are conjunctively present in some cases and disjunctively present in other cases.

## Claims

1. A device for monitoring a pulmonary system of a subject, said device comprising:
an optical member (11) for emitting a light signal through a cavity of said pulmonary system of said subject, wherein said light signal comprises at least one wavelength, wherein said at least one wavelength is within an optical window for tissue;
a detector unit (14), wherein said detector (14) is configured to be positioned on a skin surface for detecting said light signal transmitted through said cavity of said subject;
wherein said optical member (11) and said detector unit (14) are configured to be positioned so that said light signal is transmitted from said optical member (11) through said cavity to be detected by said detector (14); and
a control unit (8) for evaluating said detected light signal for determining a physiological status of said pulmonary system of said subject by evaluating a change over time in an intensity of said detected light signal transmitted through said cavity;
**characterized in that** said control unit (8) is further configured to associate said change over time in an intensity of said detected light signal transmitted through said cavity with a variation of a volume of said cavity.

2. The device of claim 1, wherein said detected light signal is associated with the same wavelength over time, such that said at least one wavelength of said emitted light signal is a single wavelength.

3. The device of any of claims 1 or 2, wherein said wavelength is not associated with an absorption band of a free gas.

4. The device of claim 1, wherein said variation of said volume is a qualitative measure.

5. The device of any of claims 1 to 4, wherein the device is further configured such that said variation in volume is detected by obtaining a first transmission signal transmitted through a first cavity, such as a first lung, and a second transmission signal transmitted through a second cavity, such as a second lung, the first and second transmission signals are then compared to detect a changing in a volume of one of said cavities in relation to the other

6. The device of any of claims 1 to 2 and 4 to 5, wherein said at least one wavelength of said emitted light signal is associated with an absorption band of a free gas in the pulmonary system.

7. The device of any of claims 1 to 6, wherein said optical member (11) is adapted to be arranged on a skin surface of the subject for emitting said light signal towards said cavity.

8. The device or any of claims 6 to 7, wherein said control unit (8) is configured to further detect said physiological status of said pulmonary system by obtaining a concentration and/or distribution of said free gas from said detected light signal transmitted through said cavity and evaluating changes in said concentration and/or distribution over time.

9. The device of any claim 8, wherein said control unit is configured to detect said physiological status of said pulmonary system by correlating said intensity of said detected light signal transmitted through said cavity and said concentration and or distribution of said free gas, over time.

10. The device of any of claims 8 to 9, wherein the light signal comprises at least two different wavelengths.

11. The device of claim 10, wherein at least one of said wavelengths is associated with an absorption band of a reference gas.

12. The device of any of claims 6 to 11, wherein the free gas is a physiological gas or a mixture of gases, such as any of oxygen, nitric oxide (NO), carbon dioxide, anaesthesia gases and water vapour.

13. The device of any of claims 1 to 12, wherein the control unit (8) is configured for controlling a medical ventilator (2) based on said detected physiological status of said pulmonary system.

## Patentansprüche

1. Vorrichtung zur Überwachung eines Lungensystems einer Person mit:
einem optischen Element (11), das ein Lichtsignal mit mindestens einer Wellenlänge durch einen Hohlraum des Lungensystems der betreffenden Person sendet, wobei die Wellenlänge innerhalb eines optischen Fensters von Gewebe liegt,
einem Detektor (14), der auf der Hautoberfläche angebracht werden kann, um das durch den Hohlraum des Lungensystems der betreffenden Person übertragene Lichtsignal nachzuweisen,
wobei das optische Element (11) und der Detektor (14) so konfiguriert sind, dass das Lichtsignal vom optischen Element (11) durch den Hohlraum übertragen wird, um vom Detektor (14) nachgewiesen zu werden, und
einer Steuereinheit (8) zur Auswertung des erfassten Lichtsignals zur Bestimmung eines physiologischen Zustands des Lungensystems der betroffenen Person durch Auswertung einer zeitlichen Änderung der Intensität des durch den Hohlraum übertragenen nachgewiesenen Lichtsignals,
mit der Eigenschaft, dass die Steuereinheit (8) ferner so konfiguriert ist, dass sie die zeitliche Änderung der Intensität des durch den Hohlraum übertragenen nachgewiesenen Lichtsignals mit einer Volumenveränderung des Hohlraums in Zusammenhang bringt.

2. Die Vorrichtung nach Anspruch 1, wobei das erfasste Lichtsignal im Zeitverlauf der gleichen Wellenlänge zugeordnet ist, sodass die mindestens eine Wellenlänge des ausgesendeten Lichtsignals eine einzelne Wellenlänge ist.

3. Die Vorrichtung nach einem der Ansprüche 1 oder 2, wobei die Wellenlänge nicht mit einem Absorptionsband eines freien Gases verbunden ist.

4. Die Vorrichtung nach Anspruch 1, wobei die Volumenveränderung eine qualitative Messgröße darstellt.

5. Die Vorrichtung nach einem der Ansprüche 1 bis 4 ist ferner so konfiguriert, dass die Volumenveränderung durch den Empfang eines ersten Transmissionssignals, das durch einen ersten Hohlraum, wie beispielsweise ein erster Lungenflügel, übertragen wird, und eines zweiten Transmissionssignals, das durch einen zweiten Hohlraum, wie beispielsweise ein zweiter Lungenflügel, übertragen wird, erfasst wird, wobei dann das erste und das zweite Transmissionssignal verglichen werden, um eine Volumenänderung einer der betreffenden Hohlräume im Verhältnis zum anderen nachzuweisen.

6. Die Vorrichtung nach einem der Ansprüche 1 bis 2 und 4 bis 5, wobei die mindestens eine Wellenlänge des ausgesendeten Lichtsignals mit einem Absorptionsband eines freien Gases im Lungensystem verbunden ist.

7. Die Vorrichtung nach einem der Ansprüche 1 bis 6, wobei das optische Element (11) auf der Hautoberfläche der Person angeordnet werden kann, um das Lichtsignal in Richtung des Hohlraums auszusenden.

8. Die Vorrichtung nach einem der Ansprüche 6 bis 7, wobei die Steuereinheit (8) ferner so ausgelegt ist, dass der physiologische Zustand des Lungensystems erfasst wird, indem eine Konzentration und/oder Verteilung des freien Gases aus dem erfassten Lichtsignal, das durch den Hohlraum übertragen wird, ermittelt und Änderungen der Konzentration und/oder Verteilung im Zeitverlauf ausgewertet werden.

9. Die Vorrichtung nach Anspruch 8, wobei die Steuereinheit so ausgelegt ist, dass der physiologische Zustand des Lungensystems durch Korrelation der Intensität des erfassten Lichtsignals, das durch den Hohlraum übertragen wird, mit der Konzentration und/oder Verteilung des freien Gases im Zeitverlauf bestimmt wird.

10. Die Vorrichtung nach einem der Ansprüche 8 bis 9, wobei das Lichtsignal mindestens zwei verschiedene Wellenlängen umfasst.

11. Die Vorrichtung nach Anspruch 10, wobei mindestens eine der Wellenlängen mit einem Absorptionsband eines Referenzgases verbunden ist.

12. Die Vorrichtung nach einem der Ansprüche 6 bis 11, wobei es sich bei dem freien Gas um ein physiologisches Gas oder ein Gemisch von Gasen handelt, wie beispielsweise Sauerstoff, Stickstoffmonoxid (NO), Kohlendioxid, Anästhesiegase und Wasserdampf.

13. Die Vorrichtung nach einem der Ansprüche 1 bis 12, wobei die Steuereinheit (8) so ausgelegt ist, dass sie ein medizinisches Beatmungsgerät (2) unter Berücksichtigung des erfassten physiologischen Zustands des Lungensystems steuert.

## Revendications

1. Dispositif de surveillance d'un système pulmonaire d'un sujet, ledit dispositif comprenant :
un élément optique (11) destiné à émettre un signal lumineux à travers une cavité dudit système pulmonaire dudit sujet, où ledit signal lumineux comprend au moins une longueur d'onde, où ladite au moins une longueur d'onde se trouve dans une fenêtre optique pour tissu ;
une unité de détecteur (14), où ledit détecteur (14) est configuré pour être positionné sur une surface cutanée afin de détecter ledit signal lumineux transmis à travers ladite cavité dudit sujet ;
où ledit élément optique (11) et ladite unité de détecteur (14) sont configurés pour être positionnés de sorte que ledit signal lumineux est transmis depuis ledit élément optique (11) à travers ladite cavité pour être détecté par ledit détecteur (14) ; et
une unité de commande (8) destinée à évaluer ledit signal lumineux détecté afin de déterminer un état physiologique dudit système pulmonaire dudit sujet en évaluant une évolution dans le temps d'une intensité dudit signal lumineux détecté transmis à travers ladite cavité ;
**caractérisé en ce que** ladite unité de commande (8) est en outre configurée pour associer ladite évolution dans le temps d'une intensité dudit signal lumineux détecté transmis à travers ladite cavité à une variation de volume de ladite cavité.

2. Dispositif selon la revendication 1, dans lequel ledit signal lumineux détecté est associé à la même longueur d'onde au fil du temps, de telle sorte que ladite au moins une longueur d'onde dudit signal lumineux émis est une seule longueur d'onde.

3. Dispositif selon l'une quelconque des revendications 1 et 2, dans lequel ladite longueur d'onde n'est pas associée à une bande d'absorption d'un gaz libre.

4. Dispositif selon la revendication 1, dans lequel ladite variation dudit volume est une mesure qualitative.

5. Dispositif selon l'une quelconque des revendications 1 à 4, où le dispositif est en outre configuré de telle sorte que ladite variation de volume est détectée en obtenant un premier signal de transmission transmis à travers une première cavité, telle qu'un premier poumon, et un second signal de transmission transmis à travers une seconde cavité, telle qu'un second poumon, les premier et second signaux de transmission sont ensuite comparés pour détecter une évolution de volume d'une desdites cavités par rapport à l'autre.

6. Dispositif selon l'une quelconque des revendications 1 à 2 et 4 à 5, dans lequel ladite au moins une longueur d'onde dudit signal lumineux émis est associée à une bande d'absorption d'un gaz libre dans le système pulmonaire.

7. Dispositif selon l'une quelconque des revendications 1 à 6, dans lequel ledit élément optique (11) est conçu pour être agencé sur une surface cutanée du sujet afin d'émettre ledit signal lumineux en direction de ladite cavité.

8. Dispositif selon l'une quelconque des revendications 6 et 7, dans lequel ladite unité de commande (8) est configurée pour détecter en outre ledit état physiologique dudit système pulmonaire en obtenant une concentration et/ou une distribution dudit gaz libre à partir dudit signal lumineux détecté transmis à travers ladite cavité et en évaluant des évolutions de ladite concentration et/ou de ladite distribution dans le temps.

9. Dispositif selon la revendication 8, dans lequel ladite unité de commande est configurée pour détecter ledit état physiologique dudit système pulmonaire en corrélant ladite intensité dudit signal lumineux détecté transmis à travers ladite cavité et ladite concentration et/ou ladite distribution dudit gaz libre, dans le temps.

10. Dispositif selon l'une quelconque des revendications 8 et 9, dans lequel le signal lumineux comprend au moins deux longueurs d'onde différentes.

11. Dispositif selon la revendication 10, dans lequel au moins une desdites longueurs d'onde est associée à une bande d'absorption d'un gaz de référence.

12. Dispositif selon l'une quelconque des revendications 6 à 11, dans lequel le gaz libre est un gaz physiologique ou un mélange de gaz, tels que l'un quelconque parmi l'oxygène, le monoxyde d'azote (NO), le dioxyde de carbone, des gaz d'anesthésie et de la vapeur d'eau.

13. Dispositif selon l'une quelconque des revendications 1 à 12, dans lequel l'unité de commande (8) est configurée pour commander un ventilateur médical (2) sur la base dudit état physiologique détecté dudit système pulmonaire.
